# EUROPEAN PATENT APPLICATION

(11) **EP 4 086 063 A1**
(43) Date of publication of application: **09.11.2022**
(21) Application number: 20910234.2
(22) Date of filing: 29.12.2020
(51) Int. Cl.: B29C 64/20, B29C 64/209, B29C 64/245, B29C 64/295, B29C 64/106, B33Y 30/00

(54) **BIOPRINTER NOZZLE, BIOPRINTER AND METHOD FOR PRINTING LUMINAL TISSUE CONSTRUCT**

(30) Priority: 30.12.2019 CN 201911389611
(71) Applicant: Revotek Co., Ltd, Sichuan 611731 (CN)
(72) Inventor: ZUO, Xiao, Chengdu, Sichuan 611731 (CN); HE, Junxuan, Chengdu, Sichuan 611731 (CN); LI, Yijun, Chengdu, Sichuan 611731 (CN); XIANG, Jie, Chengdu, Sichuan 611731 (CN); XU, Ziqing, Chengdu, Sichuan 611731 (CN); JIANG, Zhi, Chengdu, Sichuan 611731 (CN)
(74) Representative: Lavoix
(86) International application number: PCT/CN2020/141029
(87) International publication number: WO 2021/136297

(57) **Abstract**

The present disclosure provides a bioprinter nozzle, a bioprinter and a lumen tissue construct printing method. The bioprinter nozzle includes: a bio-ink flow channel disposed inside the bioprinter nozzle; a bio-ink ejection port in communication with the bio-ink flow channel and located on the lateral side of an ejection end of the bioprinter nozzle; a medical adhesive flow channel disposed inside the bioprinter nozzle and isolated from the bio-ink flow channel; and a medical adhesive ejection port in communication with the medical adhesive flow channel and disposed on the lateral side of the ejection end, the medical adhesive ejection port and the bio-ink ejection port being arranged in a staggered manner in the axial direction of the bioprinter nozzle. The bioprinter includes the bioprinter nozzle. The lumen tissue construct printing method uses the bioprinter for printing.

## Description

### Cross-Reference to Related Applications

The present disclosure is based on and claims priority to Chinese Patent Application No. 201911389611.9, filed on December 30, 2019 and entitled "Bioprinter Nozzle, Bioprinter and Lumen Tissue Construct Printing Method", the disclosure of which is hereby incorporated in its entirety.

### Field of the Invention

The present disclosure relates to the technical field of bioprinting, in particular to a bioprinter nozzle, a bioprinter and a lumen tissue construct printing method.

### Background of the Invention

A lumen tissue construct is a substitute for the body's own lumen tissue. For example, an artificial blood vessel is used as a substitute for the body's own blood vessels in the event of severe stenosis or occlusion of the body's own blood vessel.

Most of the artificial blood vessels in the prior art are made of synthetic materials such as nylon, polyester (Dacron) and polytetrafluoroethylene (PTFE). Since the inner wall of the artificial blood vessel does not have endothelial cells of a natural blood vessel, occlusion is easy to occur once more after long-term use.

Chinese patent application No. CN201910025832.1, entitled "Lumen Tissue Construct Printing Device, Printing Method Thereof and 3D Bioprinter", proposes a printing technology that can print bio-ink (including biobrick, the definition of which can make reference to patent document 201610211570.4) on the artificial lumen inner wall of the artificial lumen (e.g., an artificial blood vessel) for attachment to form a lumen tissue construct, so that the prepared lumen tissue construct has the biological performances of a real blood vessel. At present, however, it is difficult for this printing technology to prepare a lumen tissue construct greater than 10 cm in length. In order to adapt to clinical indications (e.g., arteriovenous fistula), a longer lumen tissue construct (e.g., greater than or equal to 30 cm in length) needs to be prepared.

Chinese patent application No. CN109514858A, entitled "Multi-channel 3D Printhead and Method for Manufacturing Pipe by Using Same", discloses a 3D printhead, including a plurality of extrusion pipes which are coaxial and spaced in layers from inside to outside, different printing materials being input to different extrusion pipes, and the outlet end of the inboard extrusion pipe extending out from the outlet end of the outboard extrusion pipe.

Chinese patent application No. CN107411844A, entitled "Lumen Tissue Construct and Preparation Method and Preparation Device Thereof", discloses a bioprinter 1000 for coating an outer wall of a lumen tissue construct with an adhesive, and the bioprinter 1000 has a container for containing the adhesive, a conveying channel and an adhesive outlet. In the coating, the adhesive outlet is stationary, and by moving the outer wall relative to the outlet, the adhesive is continuously squeezed out of the outlet under pressure to apply the adhesive to the inner surface of the outer wall. In this patent application, the printed inner wall formed by biological tissue is sleeved with the outer wall coated with the adhesive as described above to obtain a biological construct.

Chinese patent application No. CN105647804A, entitled "Bioprinter Nozzle and Bioprinter", discloses a coaxial two-layer flow channel nozzle of a bioprinter, wherein the first flow channel is for the passage of a primary material fluid (bio-ink), the second flow channel (i.e., outer-layer flow channel) is for the passage of an auxiliary material fluid, the inner-layer and outer-layer flow channels are reduced towards the direction of an injector outlet, thus facilitating wrapping the outflowing primary material by the auxiliary material to form a granular bioprinting material that meets the printing requirements. That is, the nozzle in this document forms micro-droplets required for printing, and cannot achieve simultaneous coating with the bio-ink and a medical adhesive.

### Summary of the Invention

An object of the present disclosure is to provide a bioprinter nozzle, a bioprinter and a lumen tissue construct printing method.

A first aspect of the present disclosure provides a bioprinter nozzle, which is provided with:
a bio-ink flow channel disposed inside the bioprinter nozzle;
a bio-ink ejection port in communication with the bio-ink flow channel and located on the lateral side of an ejection end of the bioprinter nozzle;
a medical adhesive flow channel disposed inside the bioprinter nozzle and isolated from the bio-ink flow channel; and
a medical adhesive ejection port in communication with the medical adhesive flow channel and located on the lateral side of the ejection end, the medical adhesive ejection port and the bio-ink ejection port being arranged in a staggered manner along the axial direction of the bioprinter nozzle.

In some embodiments of the present disclosure, the bio-ink ejection port is closer to the top surface of the ejection end than the medical adhesive ejection port along the axial direction of the bioprinter nozzle.

In some embodiments of the present disclosure,
the bioprinter nozzle includes a plurality of bio-ink ejection ports uniformly distributed along the circumference of the bioprinter nozzle; and/or
the bioprinter nozzle includes a plurality of the medical adhesive ejection ports uniformly distributed along the circumference of the bioprinter nozzle or the medical adhesive ejection ports include annular ports arranged around the axial direction of the bioprinter nozzle.

In some embodiments of the present disclosure, the bioprinter nozzle includes a medical adhesive scraping portion disposed on the lateral side of the bioprinter nozzle, and the medical adhesive scraping portion is located between the bio-ink ejection port and the medical adhesive ejection port along the axial direction of the bioprinter nozzle.

In some embodiments of the present disclosure, the medical adhesive scraping portion includes an annular projection or brush head disposed on the lateral side of the bioprinter nozzle.

In some embodiments of the present disclosure, the bioprinter nozzle includes:
a bio-ink nozzle body on which the bio-ink ejection port is disposed; and
a medical adhesive nozzle body disposed on the periphery of the bio-ink nozzle body, the medical adhesive ejection port being disposed on the medical adhesive nozzle body or disposed between the bio-ink nozzle body and the medical adhesive nozzle body.

In some embodiments of the present disclosure,
the bio-ink flow channel is disposed in the bio-ink nozzle body; and
the medical adhesive flow channel is disposed between the medical adhesive nozzle body and the bio-ink nozzle body.

In some embodiments of the present disclosure, the bioprinter nozzle includes a connecting fin connected between the bio-ink nozzle body and the medical adhesive nozzle body.

In some embodiments of the present disclosure,
the bio-ink flow channel is disposed in the bio-ink nozzle body;
the medical adhesive flow channel is disposed in the bio-ink nozzle body, and the side wall of the bio-ink nozzle body has a communication port in communication with the medical adhesive flow channel; and
the medical adhesive ejection port includes an annular ejection port arranged around the axial direction of the bioprinter nozzle, which is formed by the bio-ink nozzle body and the end of the medical adhesive nozzle body close to the ejection end, and the communication port is in communication with the annular ejection port.

In some embodiments of the present disclosure, the bioprinter nozzle further includes a nozzle mounting base, wherein
the bio-ink flow channel includes a first bio-ink flow channel section disposed in the bio-ink nozzle body and a second bio-ink flow channel section disposed in the nozzle mounting base, or the bio-ink flow channel is disposed in the nozzle mounting base; and
the medical adhesive flow channel includes a first medical adhesive flow channel section disposed in the medical adhesive nozzle body and a second medical adhesive flow channel section disposed in the nozzle mounting base.

In some embodiments of the present disclosure, the medical adhesive flow channel further includes a first annular buffer groove, through which the first medical adhesive flow channel section is in communication with the second medical adhesive flow channel section.

In some embodiments of the present disclosure, the first annular buffer groove is formed between the medical adhesive nozzle body and the nozzle mounting base.

In some embodiments of the present disclosure, the medical adhesive flow channel includes a second annular buffer groove disposed between the medical adhesive ejection port and the first medical adhesive flow channel section.

A second aspect of the present disclosure provides a bioprinter, including:
a bioprinter nozzle including the bioprinter nozzle as described in the first aspect of the present disclosure;
a feeding assembly connected with the bioprinter nozzle and configured to supply bio-ink to the bio-ink flow channel of the bioprinter nozzle and supply medical adhesive to the medical adhesive flow channel; and
an artificial lumen mounting assembly including an artificial lumen mounting cavity for accommodating an artificial lumen for a lumen tissue construct, the artificial lumen mounting assembly being configured to be capable of reciprocating relative to the bioprinter nozzle along the axial direction of the artificial lumen mounting cavity.

In some embodiments of the present disclosure, the feeding assembly includes:
a medical adhesive syringe configured to supply medical adhesive to the medical adhesive flow channel;
a bio-ink syringe configured to supply bio-ink to the bio-ink flow channel;
a syringe fixing assembly configured to fix the medical adhesive syringe and the bio-ink syringe; and
a push mechanism configured to be drivingly connected with a plunger of the medical adhesive syringe and a plunger of the bio-ink syringe to drive the two plungers.

In some embodiments of the present disclosure, the push mechanism includes:
a syringe plunger retaining tab provided with a notch for placing an end disc of a plunger;
a plunger retaining spring assembly including an assembly housing having a spring mounting cavity therein, a guide post and a spring, the guide post movably passing through the assembly housing and a middle part of the guide post being provided in the spring mounting cavity, a first end of the guide post being connected with the syringe plunger retaining tab, the middle part of the guide post having a protruding portion facing the radial outer side, and the spring being sleeved on the periphery of the guide post and provided between the inner wall of the spring mounting cavity close to the first end of the guide post and the protruding portion;
a spring pushing tab provided at the end of the plunger retaining spring assembly away from the plunger; and
a push-pull mechanism drivingly connected with the assembly housing, and configured to drive the assembly housing to switch a second end of the guide post and the spring pushing tab between an abutted state and a separated state; wherein in the abutted state, a space for mounting the end disc is formed between the syringe plunger retaining tab and the plunger retaining spring assembly; in the separated state, the plunger retaining spring assembly presses and fixes the end disc to the syringe plunger retaining tab; and the push-pull mechanism is configured to drive the plunger by the plunger retaining spring assembly.

In some embodiments of the present disclosure, the bioprinter includes a drive assembly, which is configured to drivingly connect with the artificial lumen mounting assembly to drive the artificial lumen mounting assembly to reciprocate along the axial direction of the artificial lumen mounting cavity.

In some embodiments of the present disclosure, the bioprinter includes a detection module configured to detect a position of the bioprinter nozzle relative to the artificial lumen mounting cavity.

In some embodiments of the present disclosure, the bioprinter includes a temperature control module configured to regulate the temperature of the artificial lumen mounting cavity.

In some embodiments of the present disclosure, the bioprinter further includes:
a support bar extending along the axial direction of the artificial lumen mounting cavity and movably disposed; and
a support plate disposed at the top end of the support bar and including a nozzle mounting port configured to be in clearance fit with the outer wall of the bioprinter nozzle to maintain the bioprinter nozzle mounted in the nozzle mounting port to be coaxial with the artificial lumen mounting cavity.

A third aspect of the present disclosure provides a lumen tissue construct printing method using the bioprinter as described in the second aspect of the present disclosure to print a lumen tissue construct, including:
filling printing material, including filling the feeding assembly with the printing material;
mounting an artificial lumen for a lumen tissue construct, including mounting the artificial lumen in artificial lumen mounting cavity of the artificial lumen mounting assembly;
initially positioning a bioprinter nozzle, including allowing the bioprinter nozzle to extend into the artificial lumen and be in an initial position; and
coating with the printing material, including moving the artificial lumen mounting assembly relative to the bioprinter nozzle along the axial direction of the artificial lumen mounting cavity, and supplying bio-ink to the bio-ink flow channel of the bioprinter nozzle and supplying medical adhesive to the medical adhesive flow channel while moving.

In some embodiments of the present disclosure, the method further includes regulating the temperature of the artificial lumen mounting cavity after mounting of the artificial lumen for the lumen tissue construct.

Based on the bioprinter nozzle having the bio-ink ejection port and the medical adhesive ejection port staggered along the axial direction of the bioprinter nozzle, provided in the present disclosure, when a lumen tissue construct is printed, the bioprinter nozzle can extend into the artificial lumen for the lumen tissue construct, and the artificial lumen mounting assembly carrying the artificial lumen for the lumen tissue construct is driven to move axially relative to the bioprinter nozzle, so that the medical adhesive is ejected at first, and along with relative movement of the bioprinter nozzle and the artificial lumen for the lumen tissue construct, the inner wall of the artificial lumen for the lumen tissue construct is coated with the medical adhesive to form a medical adhesive layer. The bio-ink is ejected within a short time interval from the time of medical adhesive ejection. Because of the adhesive effect of the medical adhesive, the bio-ink after ejection can be adhered to the medical adhesive layer immediately, which can realize simultaneous coating the inner wall of the artificial lumen for a longer lumen tissue construct with the medical adhesive and the bio-ink, thus being conductive to ensuring simultaneous formation of the medical adhesive layer and a bio-ink layer on the inner wall of the artificial lumen for the lumen tissue construct to form the desired lumen tissue construct.

The bioprinter and the lumen tissue construct printing method provided by the present disclosure have advantages corresponding to the bioprinter nozzle of the present disclosure.

Other features of the present disclosure and the advantages thereof will become clear from the following detailed description of exemplary embodiments of the present disclosure with reference to the accompanying drawings.

### Brief Description of the Drawings

Drawings illustrated herein are used for providing further understanding of the present disclosure and form part of the present disclosure, and illustrative embodiments of the present disclosure and description thereof are intended for explaining instead of improperly limiting the present disclosure. In the drawings:
Fig. 1 is a schematic perspective view of a bioprinter nozzle of an embodiment of the present disclosure.
Fig. 2 is a schematic sectional view of the bioprinter nozzle of the embodiment shown in Fig. 1 in a perspective.
Fig. 3 is a schematic sectional view of the bioprinter nozzle of the embodiment shown in Fig. 1 in another perspective.
Fig. 4 is a schematic view of an A-A section of Fig. 3.
Fig. 5 is a schematic perspective view of a bioprinter nozzle of an embodiment of the present disclosure.
Fig. 6 is a schematic sectional view of the bioprinter nozzle of the embodiment shown in Fig. 5.
Fig. 7 is a schematic perspective view of a bioprinter nozzle of an embodiment of the present disclosure.
Fig. 8 is a schematic sectional view of the bioprinter nozzle of the embodiment shown in Fig. 7.
Fig. 9 is a schematic perspective view of a bio-ink nozzle body of the bioprinter nozzle of the embodiment shown in Fig. 7.
Fig. 10 is a schematic sectional view of the bio-ink nozzle body of Fig. 9.
Fig. 11 is schematic perspective view of a medical adhesive nozzle body of the bioprinter nozzle of the embodiment shown in Fig. 7.
Fig. 12 is a schematic sectional view of the medical adhesive nozzle body of Fig. 11.
Fig. 13 is a schematic perspective view of a nozzle mounting base of the bioprinter nozzle of the embodiment shown in Fig. 7.
Fig. 14 is a schematic sectional view of the nozzle mounting base of Fig. 13.
Fig. 15 is a schematic top view of the nozzle mounting base of Fig. 13.
Fig. 16 is a schematic perspective view of a bioprinter nozzle of an embodiment of the present disclosure.
Fig. 17 is a schematic sectional view of the bioprinter nozzle of the embodiment shown in Fig. 16.
Fig. 18 is a schematic perspective view of a bio-ink nozzle body of the bioprinter nozzle of the embodiment shown in Fig. 16.
Fig. 19 is a schematic sectional view of the bio-ink nozzle body of Fig. 18.
Fig. 20 is a schematic perspective view of a medical adhesive nozzle body of the bioprinter nozzle of the embodiment shown in Fig. 16.
Fig. 21 is a schematic sectional view of the medical adhesive nozzle body of Fig. 20.
Fig. 22 is a schematic perspective view of a nozzle mounting base of the bioprinter nozzle of the embodiment shown in Fig. 16.
Fig. 23 is a schematic sectional view of the nozzle mounting base of Fig. 22.
Fig. 24 is a schematic perspective view of a bioprinter of an embodiment of the present disclosure.
Fig. 25 is a schematic perspective view of a partial structure of the bioprinter of the embodiment shown in Fig. 24.
Fig. 26 is a view of a partial structure of the bioprinter of the embodiment shown in Fig. 24.
Fig. 27 is a sectional view of a structure such as a plunger retaining spring assembly of the bioprinter of the embodiment shown in Fig. 24.

### Detailed Description of the Embodiments

The technical solutions in the embodiments of the present disclosure will be clearly and completely described below in conjunction with the accompanying drawings in the embodiments of the present invention. Apparently, embodiments described are merely part of the embodiments of the present disclosure, rather than all of the embodiments. The following description of at least one exemplary embodiment is actually merely illustrative, and in no way serves as any limitation to the present disclosure and its application or use. All other embodiments obtained by those skilled in the art based on the embodiments in the present disclosure without creative efforts fall within the protection scope of the present disclosure.

Unless otherwise specifically stated, the relative arrangement of components and steps, numerical expressions and numerical values set forth in these embodiments do not limit the scope of the present disclosure. At the same time, it should be understood that, for ease of description, the sizes of various parts shown in the drawings are not drawn to actual scale. The technologies, methods and equipment known to those of ordinary skill in related arts may not be discussed in detail, but where appropriate, the technologies, methods and equipment should be regarded as part of the granted specification. In all the examples shown and discussed herein, any specific value should be interpreted as merely exemplary, instead of limitation. Therefore, other examples of the exemplary embodiment may have different values. It should be noted that similar reference numerals and letters indicate similar items in the following drawings, so that once an item is defined in one drawing, it does not need to be further discussed in the subsequent drawings.

In the description of the present disclosure, it should be understood that the use of terms such as "first" and "second" to define parts is merely for ease of distinguishing the respective parts. Unless otherwise stated, the aforementioned terms do not have special meanings, and thus cannot be understood as limit to the protection scope of the present disclosure.

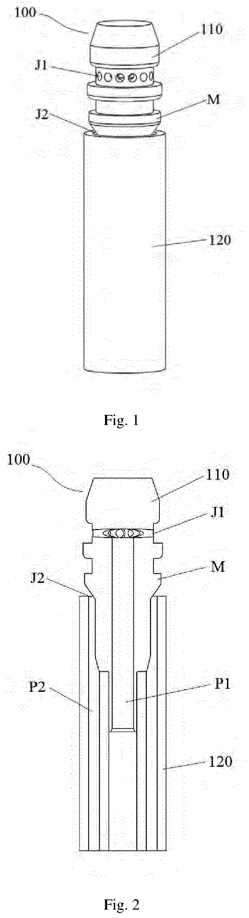

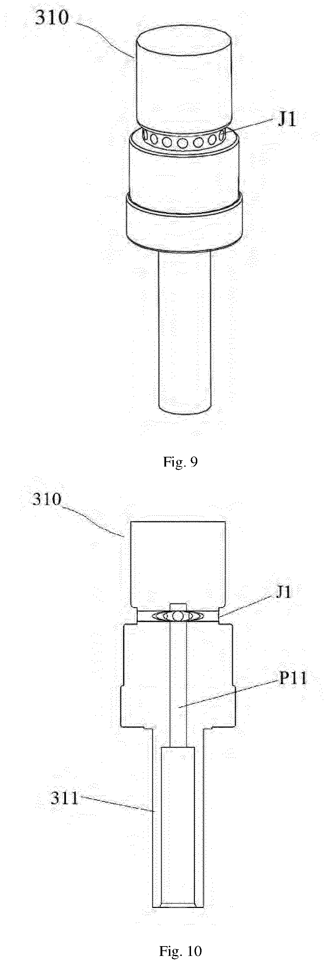

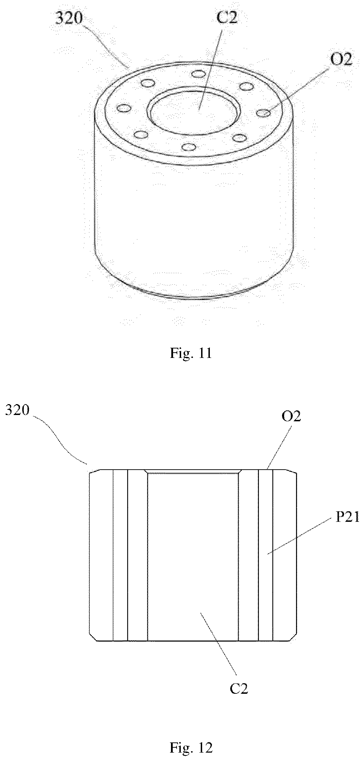

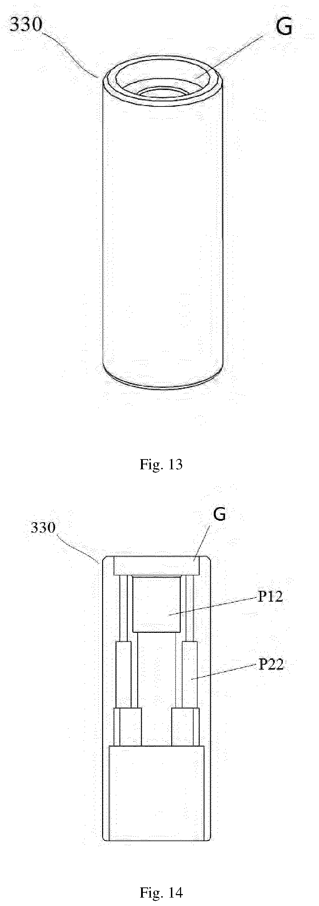

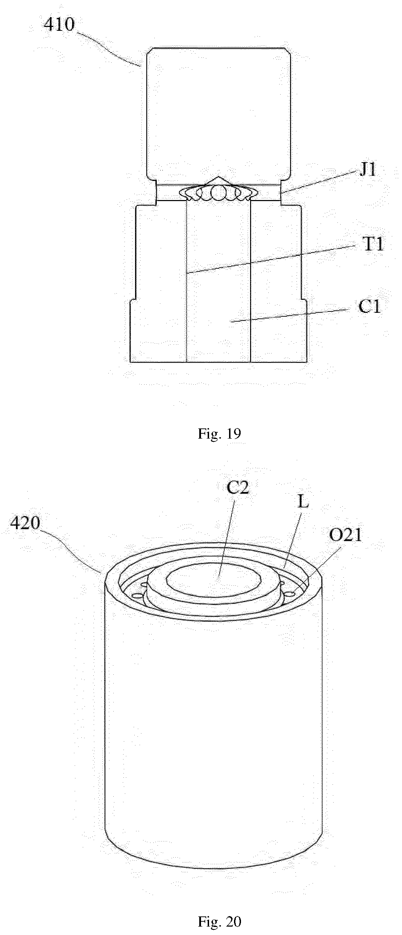

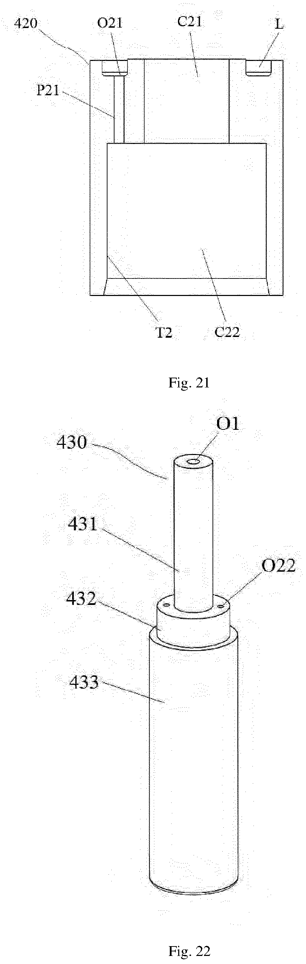

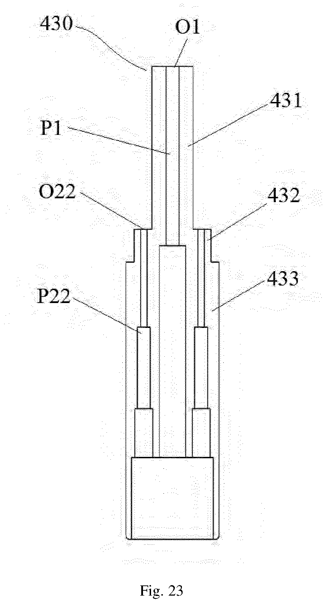

As shown in Figs. 1 to 23, some embodiments of the present disclosure provide a bioprinter nozzle. The bioprinter nozzle is provided with a bio-ink flow channel PI, a bio-ink ejection port J1, a medical adhesive flow channel P2 and a medical adhesive ejection port J2. The bio-ink flow channel P1 is disposed inside the bioprinter nozzle. The bio-ink ejection port J1 is in communication with the bio-ink flow channel P1 and located on the lateral side of an ejection end of the bioprinter nozzle. The medical adhesive flow channel P2 is disposed inside the bioprinter nozzle and isolated from the bio-ink flow channel P1. The medical adhesive ejection port J2 is in communication with the medical adhesive flow channel P2 and located on the lateral side of the ejection end of the bioprinter nozzle. In addition, the medical adhesive ejection port J2 and the bio-ink ejection port J1 are arranged in a staggered manner along the axial direction of the bioprinter nozzle.

Based on the bioprinter nozzle having the bio-ink ejection port J1 and the medical adhesive ejection port J2 staggered along the axial direction of the bioprinter nozzle, provided in the embodiments of the present disclosure, when a lumen tissue construct is printed, the bioprinter nozzle can extend into the artificial lumen for the lumen tissue construct, and the artificial lumen mounting assembly carrying the artificial lumen for the lumen tissue construct is driven to move axially relative to the bioprinter nozzle, so that the medical adhesive is ejected at first, and then along with relative movement of the bioprinter nozzle and the artificial lumen for the lumen tissue construct, and the inner wall of the artificial lumen for the lumen tissue construct is coated with the medical adhesive to form a medical adhesive layer. The bio-ink is ejected within a short time interval from the time of medical adhesive ejection. Because of the adhesive effect of the medical adhesive, the bio-ink after ejection can be adhered to the medical adhesive layer immediately, thus facilitating ensuring simultaneous formation of the medical adhesive layer and a bio-ink layer on the inner wall of the artificial lumen for the lumen tissue construct to form the desired lumen tissue construct. The bioprinter nozzle of the embodiments of the present disclosure enables simultaneous coating the inner wall of the artificial lumen for a longer lumen tissue construct with the medical adhesive and the bio-ink.

In the bioprinter nozzle of some embodiments, the bio-ink ejection port J1 is closer to the top surface of the ejection end than the medical adhesive ejection port J2 along the axial direction of the bioprinter nozzle. When the bioprinter nozzle of this structure is used to print the lumen tissue construct, the bioprinter nozzle can extend from a first end of the artificial lumen for the lumen tissue construct to a second end, and print the medical adhesive layer and the bio-ink layer on the inner wall of the artificial lumen from the second end. In the printing process, the bioprinter nozzle gradually moves from the second end of the artificial lumen to the first end, thereby being conductive to exit of the bioprinter nozzle from the artificial lumen for the lumen tissue construct while the lumen tissue construct is printed, and complete exit can be done after printing.

In the bioprinter nozzle of some embodiments, a plurality of bio-ink ejection ports J1 are uniformly distributed along the circumference of the bioprinter nozzle; and/or the medical adhesive ejection port J2 is an annular ejection port or a plurality of medical adhesive ejection ports J2 are uniformly distributed along the circumference of the bioprinter nozzle. Arrangement of the above bio-ink ejection ports J1 and the medical adhesive ejection port(s) J2 is beneficial to uniform coating with the printing material bio-ink and the medical adhesive.

In the bioprinter nozzle of some embodiments, the bioprinter nozzle includes a medical adhesive scraping portion M disposed on the lateral side of the bioprinter nozzle, and the medical adhesive scraping portion M is located between the bio-ink ejection port J1 and the medical adhesive ejection port J2 along the axial direction of the bioprinter nozzle. The medical adhesive scraping portion includes an annular projection or brush head disposed on the lateral side of the bioprinter nozzle. The medical adhesive scraping portion M is beneficial to uniform coating with the medical adhesive, and thus is beneficial to uniform and firm adhesion of the bio-ink to the inner wall of the artificial lumen.

The bioprinter nozzle of some embodiments of the present disclosure will be described in detail below in conjunction with Figs. 1 to 23.

Figs. 1 to 4 show the bioprinter nozzle 100 of an embodiment of the present disclosure. As shown in Figs. 1 to 4, the bioprinter nozzle 100 is provided with an ejection end for ejecting the printing material, the bio-ink flow channel PI, the bio-ink ejection port J1, the medical adhesive flow channel P2, and the medical adhesive ejection port J2. The bio-ink flow channel P1 is disposed inside the bioprinter nozzle 100. The bio-ink ejection port J1 is in communication with the bio-ink flow channel P1 and located on the lateral side the ejection end. The medical adhesive flow channel P2 is disposed inside the bioprinter nozzle 100 and isolated from the bio-ink flow channel P1. The medical adhesive ejection port J2 is in communication with the medical adhesive flow channel P2 and located on the lateral side of the ejection end, and the medical adhesive ejection port J2 and the bio-ink ejection port J1 are arranged in a staggered manner along the axial direction of the bioprinter nozzle 100.

The bio-ink ejection port J1 is closer to the top surface of the ejection end than the medical adhesive ejection port J2 along the axial direction of the bioprinter nozzle 100. A plurality of bio-ink ejection ports J1 are uniformly distributed along the circumference of the bioprinter nozzle 100. The medical adhesive ejection port J2 is an annular ejection port.

As shown in Figs. 1 and 2, the bioprinter nozzle 100 further includes a medical adhesive scraping portion M disposed on the lateral side of the bioprinter nozzle 100, and the medical adhesive scraping portion M is located between the bio-ink ejection ports J1 and the medical adhesive ejection port J2 along the axial direction of the bioprinter nozzle 100. The medical adhesive scraping portion M includes an annular projection disposed on the lateral side of the bioprinter nozzle. In order to facilitate coating, the side of the annular projection close to the medical adhesive ejection port J2 is inclined from the radial inner side to the radial outer side of the bioprinter nozzle 100 toward the side of the bio-ink ejection port J1.

As shown in Figs. 1 to 4, the bioprinter nozzle 100 includes a bio-ink nozzle body 110 and a medical adhesive nozzle body 120. The bio-ink ejection port J1 is disposed on the bio-ink nozzle body 110. The medical adhesive nozzle body 120 is disposed on the periphery of the bio-ink nozzle body 110. The medical adhesive ejection port J2 is disposed between the bio-ink nozzle body 110 and the medical adhesive nozzle body 120. As shown in Fig. 2, in this embodiment, the medical adhesive ejection port J2 is disposed between the circumferential outer wall of the bio-ink nozzle body 110 and the inner wall of one end of the medical adhesive nozzle body 120 close to the ejection end.

The bio-ink flow channel P1 is disposed in the bio-ink nozzle body 110. The plurality of bio-ink ejection ports J1 are in communication with the bio-ink flow channel P1.

The medical adhesive flow channel P2 is disposed between the medical adhesive nozzle body 120 and the bio-ink nozzle body 110. The medical adhesive flow channel P2 as a whole is an annular flow channel disposed on the periphery of the bio-ink flow channel PI, and the medical adhesive flows along the axial direction of the annular flow channel. A outlet (a port for discharging the medical adhesive) of the medical adhesive flow channel P2 is the medical adhesive ejection port J2.

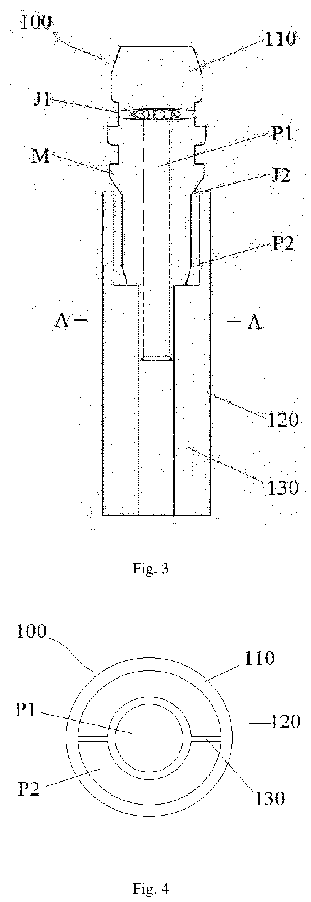

As shown in Figs. 3 to 4, the bioprinter nozzle 100 also includes a connecting fin 130 connected between the bio-ink nozzle body 110 and the medical adhesive nozzle body 120.

The bioprinter nozzle 100 of the embodiments of the present disclosure will be described in more detail below in conjunction with Figs. 1 to 4.

As shown in Figs. 1 to 4, the bioprinter nozzle 100 includes a bio-ink nozzle body 110 and a medical adhesive nozzle body 120 coaxially disposed. The bio-ink nozzle body 110 is inserted into the medical adhesive nozzle body 120. The bio-ink nozzle body 110 and the medical adhesive nozzle body 120 are fixed by the connecting fin 130. The connecting fin 130 is used to fix the bio-ink nozzle body 110 on the inner layer and the medical adhesive nozzle body 120 on the outer layer, so as to maintain the coaxiality of the bio-ink nozzle body 110 and the medical adhesive nozzle body 120.

In this embodiment, the upper end of the bio-ink nozzle body 110 is the ejection end of the bioprinter nozzle 100, and is provided with several bio-ink nozzles J1, which are uniformly disposed on the side wall of the upper end of the bio-ink nozzle body 110 along the circumference of the bioprinter nozzle 100, so that the bio-ink can be ejected from each bio-ink ejection port J1. Since the bio-ink ejection ports J1 are uniformly distributed on the side wall of the upper end of the bio-ink nozzle body 110, it is possible to enable uniform ejection of the bio-ink becoming several streams of fluid.

The bio-ink flow channel P1 is disposed inside the ink nozzle body 110, and one end (the upper end in the figure) of the bio-ink flow channel P1 is a bio-ink discharge port, and the other end (the lower end in the figure) is a bio-ink feed port. The bio-ink discharge port is in communication with the bio-ink ejection ports J1. The annular space between the inner wall of the medical adhesive nozzle body 120 and the outer wall of the bio-ink nozzle body 110 forms the medical adhesive flow channel P2, and one end (the upper end in the figure) of the medical adhesive flow channel P2 is the medical adhesive discharge port, i.e. the medical adhesive ejection port J2, and the other end (the lower end in the figure) is the medical adhesive feed port.

In order to achieve flatter medical adhesive sprayed on the inner wall of the artificial lumen for the lumen tissue construct, a medical adhesive scraping portion M is disposed in the position of the bio-ink nozzle body 110 close to the medical adhesive ejection port J2. In Figs. 1 and 2, the medical adhesive scraping portion M has an inverted cone shape.

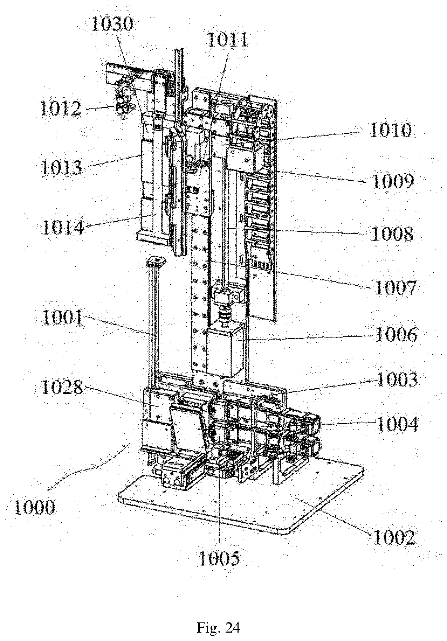

The bioprinter nozzle 100 of the embodiments of the present disclosure facilitates solving the technical problem of how to coat the inner wall of a longer lumen tissue construct with the medical adhesive and the bio-ink simultaneously. At the start of coating, the bioprinter nozzle 100 first extends to the interior of the artificial lumen for the lumen tissue construct, and the position of the medical adhesive ejection port J2 is aligned with the upper edge of the artificial lumen for the lumen tissue construct. With reference to the bioprinter 1000 shown in Figs. 24 to 26, at this point, the feeding assembly 1005 of the bioprinter 1000 starts to provide medical adhesive and bio-ink, and the drive assembly 1009 of the printer 1000 drives the artificial lumen for the lumen tissue construct to move upward relative to the bioprinter nozzle 100. During the movement, as the bio-ink nozzle J1 and the medical adhesive nozzle J2 are staggered in position, the inner wall of the artificial lumen can be coated with the medical adhesive after ejection first. Along with the relative movements of the bioprinter nozzle 100 and the artificial lumen, the bio-ink is ejected to continue coating the medical adhesive layer, and at the same time, it will be able to immediately adhere the bio-ink to the medical adhesive layer due to the adhesive effect of the medical adhesive. When the bioprinter nozzle 100 completely exits from the artificial lumen, it is possible to form the medical adhesive layer and the bio-ink layer simultaneously on the inner wall of the artificial lumen, so as to form the desired lumen tissue construct.

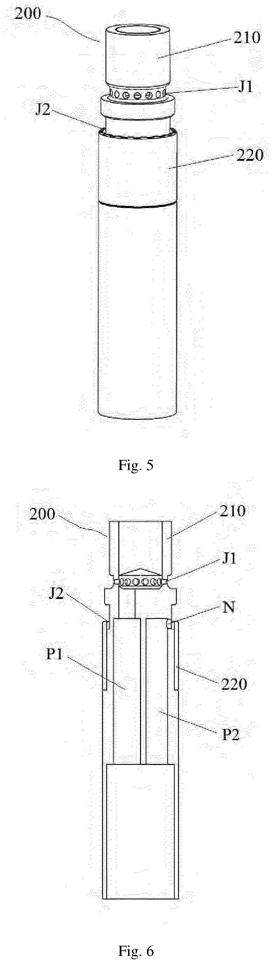

Figs. 5 and 6 show the bioprinter nozzle 200 of an embodiment of the present disclosure. As shown in Figs. 5 and 6, the bioprinter nozzle 200 is provided with an ejection end for ejecting the printing material, the bio-ink flow channel P1, the bio-ink ejection port J1, the medical adhesive flow channel P2 and the medical adhesive ejection port J2. The bio-ink flow channel P1 is disposed inside the bioprinter nozzle 200. The bio-ink ejection port J1 is in communication with the bio-ink flow channel P1 and located on the lateral side the ejection end. The medical adhesive flow channel P2 is disposed inside the bioprinter nozzle 200 and isolated from the bio-ink flow channel P1. The medical adhesive ejection port J2 is in communication with the medical adhesive flow channel P2 and located on the lateral side of the ejection end, and the medical adhesive ejection port J2 and the bio-ink ejection port J1 are arranged in a staggered manner along the axial direction of the bioprinter nozzle 200.

The bio-ink ejection port J1 is closer to the top surface of the ejection end than the medical adhesive ejection port J2 along the axial direction of the bioprinter nozzle 200. A plurality of bio-ink ejection ports J1 are uniformly distributed along the circumference of the bioprinter nozzle 200. The medical adhesive ejection port J2 is an annular ejection port.

As shown in Figs. 5 and 6, the bioprinter nozzle 200 includes a bio-ink nozzle body 210 and a medical adhesive nozzle body 220. The bio-ink ejection port J1 is disposed on the bio-ink nozzle body 210. The medical adhesive nozzle body 220 is disposed on the periphery of the bio-ink nozzle body 210.

The bio-ink flow channel P1 is disposed in the bio-ink nozzle body 210. The plurality of bio-ink ejection ports J1 are in communication with the bio-ink flow channel P1.

The medical adhesive flow channel P2 is disposed in the bio-ink nozzle body 210, and the side wall of the bio-ink nozzle body 210 is provided with a communication port N in communication with the medical adhesive flow channel P2.

The medical adhesive ejection port J2 is disposed between the bio-ink nozzle body 210 and the medical adhesive nozzle body 220. The medical adhesive ejection port J2 includes an annular ejection port arranged around the axial direction of the bioprinter nozzle, the annular ejection port is formed by the bio-ink nozzle body 210 and the end of the medical adhesive nozzle body 220 close to the ejection end, and the communication port N is in communication with the annular ejection port. As shown in Fig. 6, in this embodiment, the medical adhesive ejection port J2 is disposed between the circumferential outer wall of the bio-ink nozzle body 210 and the inner wall of one end of the medical adhesive nozzle body 220 close to the ejection end.

The bioprinter nozzle 200 of the embodiments of the present disclosure will be described in more detail below in conjunction with Figs. 5 and 6.

As shown in Figs. 5 and 6, the bioprinter nozzle 200 includes a bio-ink nozzle body 210 and a medical adhesive nozzle body 220. The bio-ink flow channel P1 and the medical adhesive flow channel P2 are disposed in parallel inside the bio-ink nozzle body 210. One end (the upper end in the figure) of the bio-ink flow channel P1 is a bio-ink discharge port, and the other end (the lower end in the figure) is a bio-ink feed port. One end (the upper end in the figure) of the medical adhesive flow channel P2 is a medical adhesive discharge port, and the other end (the lower end in the figure) is a medical adhesive feed port. The upper end of the bio-ink nozzle body 210 is provided with several bio-ink ports J1, which are uniformly disposed on the side wall of the upper end of the bio-ink nozzle body 210 along the circumference of the bioprinter nozzle 200, and the bio-ink ejection ports J1 are in communication with the bio-ink discharge port. The middle part of the bio-ink nozzle body 210 is provided with a step, on which a sleeve-shaped medical adhesive nozzle body 220 is sleeved to the bio-ink nozzle body 210. An annular ejection port between the medical adhesive nozzle body 220 and the outer wall of the bio-ink nozzle body 210 is the medical adhesive ejection port J2. The medical adhesive ejection port J2 is in communication with the medical adhesive discharge port of the medical adhesive flow channel P2.

The working principle of the bioprinter nozzle 200 is the same as that of the bioprinter nozzle 100, and will not be repeated here.

In the bioprinter nozzle 200, it can set the diameters of the bio-ink flow channel P1 and the adhesive flow channel P2 to be smaller than that of the corresponding flow channel of the bioprinter nozzle 100, so that the usage amounts of the bio-ink and the medical adhesive can be saved in the printing process.

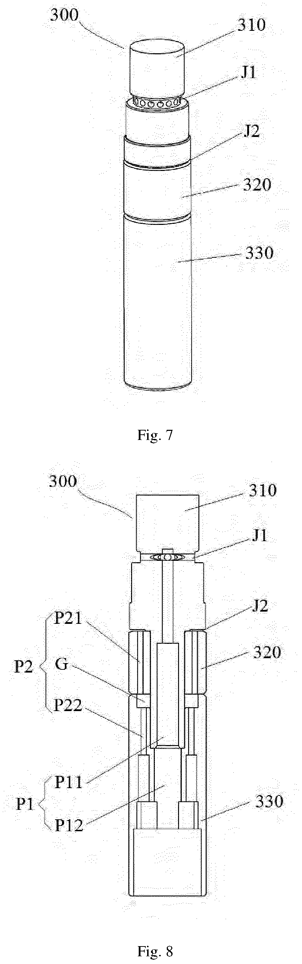

Figs. 7 to 15 show the bioprinter nozzle 300 of an embodiment of the present disclosure. As shown in Figs. 7 to 15, the bioprinter nozzle 300 is provided with an ejection end (the upper end in the figure) for ejecting the printing material, the bio-ink flow channel PI, the bio-ink ejection port J1, the medical adhesive flow channel P2 and the medical adhesive ejection port J2.

The bio-ink flow channel P1 is disposed inside the bioprinter nozzle 300. The bio-ink ejection port J1 is in communication with the bio-ink flow channel P1 and located on the lateral side the ejection end. The medical adhesive flow channel P2 is disposed inside the bioprinter nozzle 300 and isolated from the bio-ink flow channel P1. The medical adhesive ejection port J2 is in communication with the medical adhesive flow channel P2 and located on the lateral side of the ejection end, and the medical adhesive ejection port J2 and the bio-ink ejection port J1 are arranged in a staggered manner along the axial direction of the bioprinter nozzle 300.

The bio-ink ejection port J1 is closer to the top surface of the ejection end than the medical adhesive ejection port J2 along the axial direction of the bioprinter nozzle 300. A plurality of bio-ink ejection ports J1 are uniformly distributed along the circumference of the bioprinter nozzle 300. The medical adhesive ejection port J2 is an annular ejection port.

As shown in Figs. 7 to 15, the bioprinter nozzle 300 includes a bio-ink nozzle body 310, a medical adhesive nozzle body 320 and a nozzle mounting base 330. The bio-ink nozzle body 310 and the medical adhesive nozzle body 320 are disposed on the nozzle mounting base 330.

The bio-ink ejection port J1 is disposed on the bio-ink nozzle body 310. The medical adhesive nozzle body 320 has a sleeve-shaped structure and is disposed on the periphery of the bio-ink nozzle body 310.

The medical adhesive ejection port J2 includes an annular ejection port, which is formed by the bio-ink nozzle body 310 and the end of the medical adhesive nozzle body 320 close to the ejection end, and the communication port is in communication with the annular ejection port. As shown in Fig. 8, the medical adhesive ejection port J2 is disposed between the bottom wall of the protruding proportion in the middle part of the bio-ink nozzle body 310 and the end wall of the medical adhesive nozzle body 320 close to the ejection end of the bioprinter nozzle 300.

The bio-ink flow channel P1 includes a first bio-ink flow channel section P11 disposed in the bio-ink nozzle body 310 and a second bio-ink flow channel section P12 disposed in the nozzle mounting base 300.

The medical adhesive flow channel P2 includes a first medical adhesive flow channel section P21 disposed in the medical adhesive nozzle body 320 and a second medical adhesive flow channel section P22 disposed in the nozzle mounting base. As shown in Fig. 8, the medical adhesive flow channel P2 further includes a first annular buffer groove G, through which the first medical adhesive flow channel section P21 is in communication with the second medical adhesive flow channel section P22. As shown in Fig. 8, the first annular buffer groove G is formed between the medical adhesive nozzle body 320 and the nozzle mounting base 330.

The bioprinter nozzle 300 of the embodiments of the present disclosure will be further described below in conjunction with Figs. 7 to 15.

As shown in Figs. 7 to 15, the bioprinter nozzle 300 includes a bio-ink nozzle body 310, a medical adhesive nozzle body 320 and a nozzle mounting base 330. The nozzle mounting base 330 is sequentially sleeved with the medical adhesive nozzle body 320 and the bio-ink nozzle body 310, and the bio-ink nozzle body 310 passes through the medical adhesive nozzle body 320 and is inserted into the nozzle mounting base 330.

The nozzle mounting base 330 is provided therein with a second bio-ink flow channel section P12, with one end thereof being the feed port of the second bio-ink flow channel section P12, and the other end being the discharge port of the second bio-ink flow channel section P12. Second medical adhesive flow channel sections P22 are disposed symmetrically on both sides of the second bio-ink flow channel section P12. In the embodiments not shown, in order to save the usage amount of the medical adhesive, it can set the number of the second medical adhesive flow channel section P22 to be one. One end of the second medical adhesive flow channel section P22 is the discharge port of the second medical adhesive flow channel section P22, and the other end is the feed port of the second medical adhesive flow channel section P22.

The bio-ink nozzle body 310 includes a mounting column 311 disposed at the lower part, and the middle part of the medical adhesive nozzle body 320 is provided with a mounting cavity C2 for penetration of the mounting column 311 of the bio-ink nozzle body 310 therefrom. A plurality of first medical adhesive flow channel sections P21 are uniformly disposed outside of the mounting cavity C2. One end of the first medical adhesive flow channel section P21 is a first medical adhesive discharge port O2, and the other end is a first medical adhesive feed port. The upper end surface of the medical adhesive nozzle body 320 is provided with several first medical adhesive discharge ports O2 uniformly disposed along the circumferential direction. In addition, the first medical adhesive discharge port O2 (as a communication port) is in communication with the medical adhesive ejection port J2 (an annular ejection port), and the first medical adhesive feed port is in communication with a second medical adhesive discharge port.

As shown in Fig. 8, the second medical adhesive flow channel sections P22 are disposed symmetrically on both sides of the second bio-ink flow channel section P12, and the first annular buffer groove G is disposed above the second medical adhesive flow channel section P22. One end of the second medical adhesive flow channel section P22 is the second medical adhesive discharge port, and the other end is the second medical adhesive feed port. The second medical adhesive discharge port is in communication with the first annular buffer groove G. The middle part of the medical adhesive nozzle body 320 is provided with a mounting cavity C2, several first medical adhesive flow channel sections P21 are uniformly provided on the periphery of the mounting cavity C2. One end of the first medical adhesive flow channel sections P21 is a first medical adhesive discharge port O2 in communication with the medical adhesive ejection port, and the other end is the first medical adhesive feed port in communication with the first annular buffer groove G.

The upper end of the bio-ink nozzle body 310 is provided with several bio-ink ejection ports J1, which are uniformly disposed on the side wall of the upper end of the bio-ink nozzle body 310. The bio-ink nozzle body 310 is provided with a mounting column 311 at the lower end, and provided therein with a first bio-ink flow channel section P11, with one end of the first bio-ink flow channel section P11 being the first bio-ink discharge port in communication with the bio-ink ejection port J1, and the other end being the bio-ink feed port in communication with the second bio-ink discharge port.

The working principle of the bioprinter nozzle 300 is the same as that of the bioprinter nozzle 100, and will not be repeated here.

In the bioprinter nozzle 300, the diameters of the bio-ink flow channel and the medical adhesive flow channel become smaller than that of the bioprinter nozzle 100, so that the usage amounts of the bio-ink and the medical adhesive are saved in the printing process.

In the bioprinter nozzle 300, the upper end of the nozzle mounting base 330 and the lower end of the mounting column 311 may be a threaded connection structure, which facilitates assembly and disassembly of the nozzle mounting base 330, the bio-ink nozzle body 310 and the medical adhesive nozzle body 320.

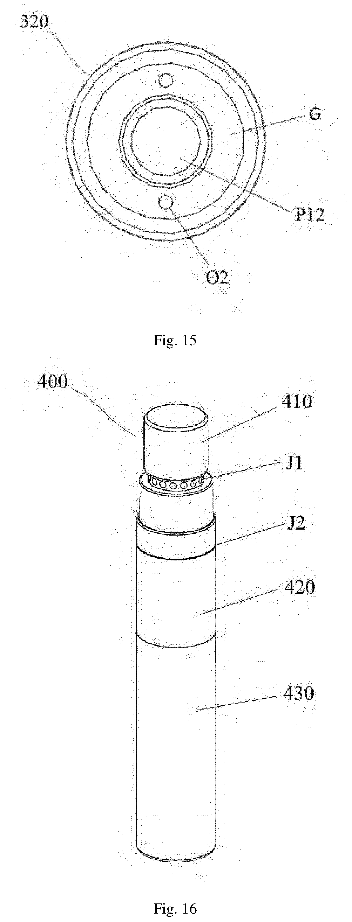

Figs. 16 to 23 show the bioprinter nozzle 400 of some embodiments of the present disclosure. As shown in Figs. 16 to 23, the bioprinter nozzle 400 is provided with an ejection end (the upper end in the figure) for ejecting the printing material, the bio-ink flow channel PI, the bio-ink ejection port J1, the medical adhesive flow channel P2 and the medical adhesive ejection port J2.

The bio-ink flow channel P1 is disposed inside the bioprinter nozzle 400. The bio-ink ejection port J1 is in communication with the bio-ink flow channel P1 and located on the lateral side the ejection end. The medical adhesive flow channel P2 is disposed inside the bioprinter nozzle 400 and isolated from the bio-ink flow channel P1. The medical adhesive ejection port J2 is in communication with the medical adhesive flow channel P2 and located on the lateral side of the ejection end, and the medical adhesive ejection port J2 and the bio-ink ejection port J1 are arranged in a staggered manner along the axial direction of the bioprinter nozzle 400.

The bio-ink ejection port J1 is closer to the top surface of the ejection end than the medical adhesive ejection port J2 along the axial direction of the bioprinter nozzle 400. A plurality of bio-ink ejection ports J1 are uniformly distributed along the circumference of the bioprinter nozzle 400, and the medical adhesive ejection port J2 is an annular ejection port.

As shown in Figs. 16 to 23, the bioprinter nozzle includes a bio-ink nozzle body 410, a medical adhesive nozzle body 420 and a nozzle mounting base 430. The bio-ink nozzle body 410 and the medical adhesive nozzle body 420 are disposed on the nozzle mounting base 430.

The bio-ink ejection port J1 is disposed on the bio-ink nozzle body 410. The medical adhesive nozzle body 420 has a sleeve-shaped structure and is disposed on the periphery of the bio-ink nozzle body 410.

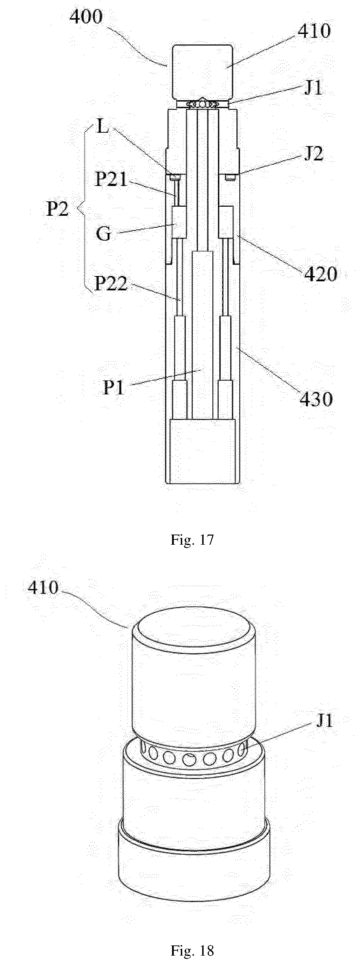

The medical adhesive ejection port J2 includes an annular ejection port, which is formed by the bio-ink nozzle body 410 and the end of the medical adhesive nozzle body 420 close to the ejection end. As shown in Fig. 17, the medical adhesive ejection port J2 is disposed between the bottom wall of the protruding proportion in the middle part of the bio-ink nozzle body 410 and the end wall of the medical adhesive nozzle body 420 close to the ejection end of the bioprinter nozzle 400.

The bio-ink flow channel P1 is disposed in the nozzle mounting base 430.

The medical adhesive flow channel P2 includes a first medical adhesive flow channel section P21 disposed in the medical adhesive nozzle body 420 and a second medical adhesive flow channel section P22 disposed in the nozzle mounting base 430. As shown in Fig. 17, the medical adhesive flow channel P2 further includes a first annular buffer groove G, through which the first medical adhesive flow channel section P21 is in communication with the second medical adhesive flow channel section P22. As shown in Fig. 17, the first annular buffer groove G is formed between the medical adhesive nozzle body 420 and the nozzle mounting base 430.

As shown in Fig. 17, the medical adhesive flow channel P2 includes a second annular buffer groove L disposed between the medical adhesive ejection port J2 and the first medical adhesive flow channel section P21.

The bioprinter nozzle 400 of the embodiments of the present disclosure will be further described below in conjunction with Figs. 16 to 23.

As shown in Figs. 16 to 23, the bioprinter nozzle 400 includes a bio-ink nozzle body 410, a medical adhesive nozzle body 420 and a nozzle mounting base 430. The nozzle mounting base 430 is sequentially sleeved with the medical adhesive nozzle body 420 and the bio-ink nozzle body 410.

The nozzle mounting base 430 is provided therein with a bio-ink flow channel PI, with one end (the upper end in the figure) of the bio-ink flow channel P1 being the bio-ink discharge port O1, and the other end (the lower end in the figure) being the bio-ink feed port.

Second medical adhesive flow channel sections P22 are disposed symmetrically respectively on both sides of the bio-ink flow channel P1 in the nozzle mounting base 430. One end of the second medical adhesive flow channel section P22 is the second medical adhesive discharge port O22, and the other end is the second medical adhesive feed port. In some embodiments not shown, in order to save the usage amount of the medical adhesive, it can set the number of the second medical adhesive flow channel section P22 to be one.

The nozzle mounting base 430 is provided with a first mounting column 431 for mounting the bio-ink nozzle body 410, a second mounting column 432 for mounting the medical adhesive nozzle body 420, and a mounting base body 433 from top to bottom respectively. The outer wall upper portion of the first mounting column 431 is provided with a first external thread, and the outer wall of the second mounting column 432 is provided with a second external thread. The second medical adhesive discharge port O22 is located on the upper end surface of the second mounting column 432. The bio-ink discharge port O1 is located on the upper end surface of the first mounting column 431.

A first mounting cavity C21 for passing the first mounting column 431 and a second mounting cavity C22 for passing the second mounting column 432 are sequentially disposed inside the medical adhesive nozzle body 420. The inner wall of the second mounting cavity C22 is provided with a second internal thread T2, and detachable connection between the medical adhesive nozzle body 420 and the nozzle mounting base 430 is achieved through thread fit of the second internal thread T2 and the second external thread.

The upper end surface of the medical adhesive nozzle body 420 is provided with a second annular buffer groove L, the bottom surface of which is provided with several first medical adhesive discharge ports 021. The first medical discharge ports 021 are uniformly distributed on the bottom surface of the second annular buffer groove L. Several first medical adhesive flow channel sections P21 are disposed below the second annular buffer groove L in a manner of corresponding to the several medical adhesive discharge ports 021. One end (the upper end in the figure) of the first medical adhesive flow channel section P21 is the first medical adhesive discharge port 021, and the other end (the lower end in the figure) is the first medical adhesive feed port. The first medical adhesive discharge port 021 is in communication with the medical adhesive ejection port J2. The first medical adhesive feed port is in communication with the second medical adhesive discharge port O22.

The bio-ink nozzle body 410 is provided therein with a mounting cavity C1 for fitting with and mounting the first mounting column 431. The inner wall of the mounting cavity C1 is provided with a first internal thread T1. Detachable connection between the bio-ink nozzle body 410 and the nozzle mounting base 430 is achieved through thread fit of the first internal thread T1 and the first external thread.

The medical adhesive nozzle body 420 and the bio-ink nozzle body 410 are detachably connected with the nozzle mounting base 430 respectively, which can facilitate disassembling and cleaning the medical adhesive nozzle body 420 and the bio-ink nozzle body 410.

The upper end of the bio-ink nozzle body 410 is provided with several bio-ink nozzles J1, which are uniformly disposed on the side wall of the upper end of the bio-ink nozzle body 410 along the circumference, and the bio-ink ejection ports J1 are in communication with the bio-ink discharge port O1.

In order to coat the inner wall of the artificial lumen for the lumen tissue construct with the medical adhesive ejected from the medical adhesive ejection port J2 more uniformly, a sponge brush head is disposed at a gap between the lower end of the bio-ink nozzle body 410 and the upper end of the medical adhesive nozzle body 420. It is possible to first moisten the sponge brush head with the medical adhesive ejected from the medical adhesive ejection port J2, wet the sponge brush head, and then uniformly coat the inner wall of the artificial lumen for the lumen tissue construct with the medical adhesive by contact and extrusion between the sponge brush head and the inner wall of the artificial lumen for the lumen tissue construct.

The working principle of the bioprinter nozzle 400 is the same as that of the bioprinter nozzle 100, and will not be repeated here.

In the bioprinter nozzle 400, the diameters of the bio-ink flow channel P1 and the medical adhesive flow channel P2 become smaller than that of the corresponding flow channel of the bioprinter nozzle 100, so the usage amounts of the bio-ink and the medical adhesive can be saved in the printing process. By changing the structure of the medical adhesive ejection port J2 in the medical adhesive nozzle body 420 and adding the sponge brush head, it is possible to coat the inner wall of the artificial lumen for the lumen tissue construct more uniformly in the coating process with the medical adhesive.

As shown in Figs. 24 to 26, some embodiment of the present disclosure provide a bioprinter 1000. The bioprinter 1000 of the embodiments of the present disclosure mainly includes a bioprinter nozzle 1001, a feeding assembly 1005 and an artificial lumen mounting assembly 1030.

The bioprinter nozzle 1001 includes the bioprinter nozzle of the embodiments described above. For example, the bioprinter nozzle 1001 can be the bioprinter nozzle 100, 200, 300 or 400 described above or the variation thereof.

The feeding assembly 1005 is connected with the bioprinter nozzle 1001, and configured to supply bio-ink to the bio-ink flow channel P1 of the bioprinter nozzle 1001 and supply medical adhesive to the medical adhesive flow channel P2.

The artificial lumen mounting assembly 1030 includes an artificial lumen mounting cavity 1014 for accommodating the artificial lumen for the lumen tissue construct. The artificial lumen mounting assembly 1030 is configured to be capable of reciprocating relative to the bioprinter nozzle 1001 along the axial direction of the artificial lumen mounting cavity 1014. The artificial lumen, for example, is an artificial blood vessel, an artificial bile vessel, an artificial urinary catheter.

In the bioprinter 1000 of some embodiments, the feeding assembly 1005 primarily includes a medical adhesive syringe 1003, a bio-ink syringe 1004, a syringe fixing assembly, and a push mechanism. The medical adhesive syringe 1003 is configured to supply medical adhesive to the medical adhesive flow channel P2. The bio-ink syringe 1004 is configured to supply bio-ink to the bio-ink flow channel P1. The syringe fixing assembly is configured to fix the medical adhesive syringe 1003 and the bio-ink syringe 1004. The push mechanism is configured to be drivingly connected with a plunger of the medical adhesive syringe 1003 and a plunger of the bio-ink syringe 1004 to drive the two plungers.

In the bioprinter 1000 of some embodiments, the push mechanism primarily includes a syringe plunger retaining tab 1024, a plunger retaining spring assembly 1029, a spring pushing tab 1025, and a push-pull mechanism 1026.

The syringe plunger retaining tab 1024 is provided with a notch for placing an end disc of the plunger.

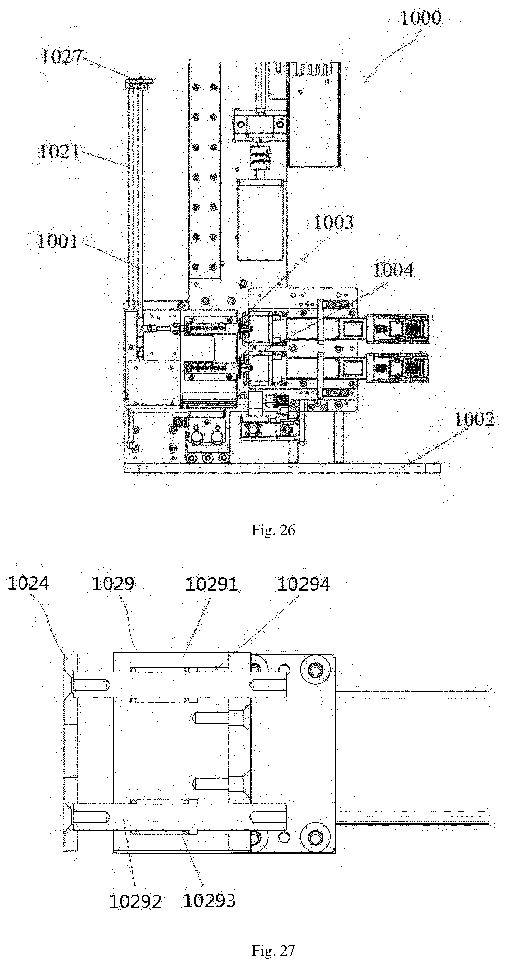

As shown in Fig. 27, the plunger retaining spring assembly 1029 includes an assembly housing 10291, a guide post 10292, and a spring 10293. The assembly housing 10291 has a spring mounting cavity 10294 therein. The guide post 10292 movably passes through the assembly housing 10291 and has a middle part located in the spring mounting cavity 10294. The first end of the guide post 10292 is connected with the syringe plunger retaining tab 1024. The middle part of the guide post 10292 has a protruding portion facing the radial outer side, and the spring 10293 is sleeved on the periphery of the guide post 10292 and provided between the inner wall of the spring mounting cavity 10294 close to the first end of the guide post 10292 and the protruding portion.

The spring pushing tab 1025 is provided at an end of the plunger retaining spring assembly 1029 away from the plunger.

The push-pull mechanism 1026 is connected with the assembly housing 10291 in a driving manner, and is configured to drive the assembly housing 10291 to move to switch the second end of the guide post 10292 and the spring pushing tab 1025 between an abutted state and a separated state. In the abutted state, a space for mounting the end disc is formed between the syringe plunger retaining tab 1024 and the plunger retaining spring assembly 1029. In the separated state, the plunger retaining spring assembly 1029 presses and fixes the end disc to the syringe plunger retaining tab 1024, and the push-pull mechanism 1026 is configured to drive the plunger through the plunger retaining spring assembly 1029.

In some embodiments, the bioprinter 1000 includes a drive assembly 1009. The drive assembly 1009 is connected with the artificial lumen mounting assembly 1030 in a driving manner and configured to drive the artificial lumen mounting assembly 1030 to reciprocate along the axial direction of the artificial lumen mounting cavity 1014.

In some embodiments, the bioprinter 1000 further includes a detection module 1012. The detection module 1012 is configured to detect relative positions of the bioprinter nozzle 1001 and the artificial lumen mounting cavity 1014.

In some embodiments, the bioprinter includes a temperature control module 1013. The temperature control module 1013 is configured to regulate the temperature of the artificial lumen mounting cavity 1014, while the temperature control module 1013 may also be separately disposed on a syringe pressing plate 1018 and a pipe clamping block 1028, respectively, so as to achieve temperature regulation of the medical adhesive syringe 1003, the bio-ink syringe 1004, the bio-ink connection pipe 1015, and the medical adhesive connection pipe 1016.

In some embodiments, the bioprinter includes a support bar 1021 and a support plate 1027. The support bar 1021 extends along the axial direction of the artificial lumen mounting cavity 1014 and movably disposed. The support plate 1027 is disposed at the top end of the support bar 1021; and the support plate 1027 includes a nozzle mounting port configured to be in clearance fit with the outer wall of the bioprinter nozzle 1001 to maintain the bioprinter nozzle 1001 mounted in the nozzle mounting port to be coaxial with the artificial lumen mounting cavity 1014.

The bioprinter 1000 in embodiments of the present disclosure will be described in more detail below in conjunction with Figs. 24 to 26. The term "front" as referred when describing the bioprinter 1000 in the embodiments of the present disclosure refers to the movement direction of the plunger of the syringe of the bioprinter 1000 when the bio-ink and the medical adhesive are delivered outward, and the term "back" refers to the direction opposite to the movement direction. In Figs. 24 to 26, "front" is on the left and "back" is on the right.

As shown in Figs. 24 to 26, the bioprinter 1000 includes a mounting base 1002, and a bioprinter nozzle 1001, a feeding assembly 1005, an artificial lumen mounting assembly 1030, a drive assembly 1009, and a detection module 1012 disposed on the mounting base 1002.

The feeding assembly 1005 includes a medical adhesive syringe 1003, a bio-ink syringe 1004, a syringe mounting plate 1017, a syringe pressing assembly, a pressing plate drive mechanism and a push mechanism.

The medical adhesive syringe 1003 and the bio-ink syringe 1004 are both mounted to the syringe mounting plate 1017. The syringe pressing assembly includes a syringe pressing plate 1018, a rotating shaft, and a gear rack assembly. The syringe pressing plate 1018 is fixedly connected with the rotating shaft, which is fixedly connected with the gear. The syringe pressing plate 1018 is driven by cooperation of the rotating shaft and the gear and rack. The gear connected with the rotating shaft drives the rotating shaft to rotate when moving on the rack, thereby moving the syringe pressing plate 1018 and pressing the two syringes on the syringe mounting plate 1017.

A push mechanism is disposed at the rear of each of the two syringes, and includes a plunger fixing mechanism disposed at the front end and the push-pull mechanism 1026 disposed at the rear end.

The plunger fixing mechanism includes a syringe plunger retaining tab 1024, a plunger retaining spring assembly 1029 and a spring pushing tab 1025.

The syringe plunger retaining tab 1024 is provided with a notch for placing an end disc of the plunger. The syringe plunger retaining tab 1024 is provided in front of the plunger retaining spring assembly 1029 and connected with the later. The spring pushing tab 1025 is provided at the rear of the plunger retaining spring assembly 1029.

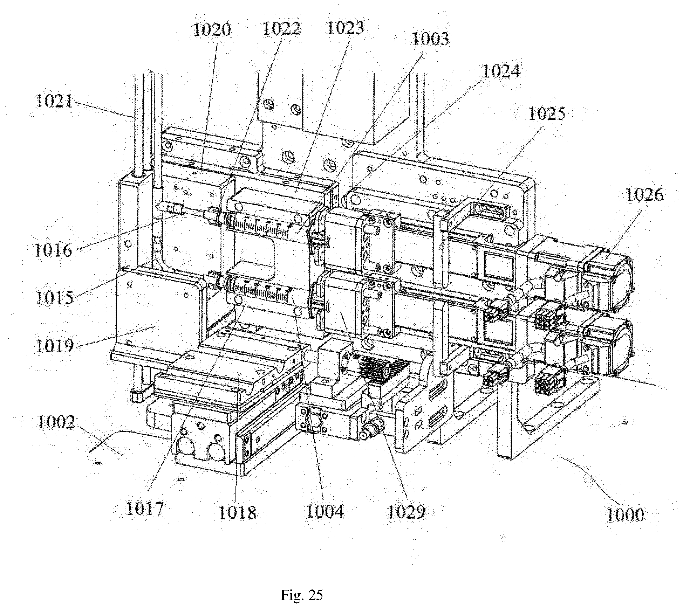

As shown in Fig. 25, the plunger retaining spring assembly 1029 has a block structure as a whole. The syringe plunger retaining tab 1024 includes an assembly housing 10291 and two (upper and lower) guide posts 10292 disposed on the assembly housing 10291 and two springs 10293 disposed in the two (upper and lower) spring mounting cavities 10294 of the assembly housing 10291 respectively. Each spring 10293 is provided in the corresponding spring mounting cavity 10294 and sleeved on the periphery of the corresponding guide post 10292, and the spring 10293 is provided between the protruding portion of the corresponding guide post 10292 and the inner wall of the spring mounting cavity 10294 close to the first end of the guide post 10292. The two ends of the guide posts 10292 extend out from the assembly housing 10291. The front end (the first end) of the guide post 10292 is connected with the syringe plunger retaining tab 1024.

The push-pull mechanism 1026 is connected with the assembly housing 10291 in a driving manner. When the push-pull mechanism 1026 drives the plunger retaining spring assembly 1029 to move backward, the rear end (the second end) of the guide post 10292 and the spring pushing tab 1025 are in an abutted state, which facilitates forward movement of the guide post 10292 to increase the spacing between the syringe plunger retaining tab 1024 and the assembly housing 10291 of the plunger retaining spring assembly 1029, thereby leaving a space for placing the end disc of the plunger. After the rear end (the second end) of the guide post 10292 of the plunger retaining spring assembly 1029 leaves the spring pushing tab 1025, i.e., when they are in the separated state, the guide post 10292 moves relative to the assembly housing 10291 toward its rear end by resilience of the spring 10293 to reduce the spacing between the syringe plunger retaining tab 1024 and the assembly housing 10291 of the plunger retaining spring assembly 1029 and to press and fix the end disc of the plunger, thus completing the mounting of the plunger retaining spring assembly 1029 and the plunger of the syringe.

Then the plunger retaining spring assembly 1029 can be driven by the push-pull mechanism 1026 to move back and forth, thus driving the plunger of the syringe to squeeze out the bio-ink or medical adhesive in the syringe, so that the feeding assembly 1005 completes feeding to the bioprinter nozzle 1001. Preferably, the plunger retaining spring assembly 1029 is disposed on the guide rail 1031, and reciprocates along the guide rail 1031.

The front end of the medical adhesive syringe 1003 is connected to the medical adhesive flow channel of the bioprinter nozzle 1001 through the medical adhesive connection pipe 1016. The front end of the bio-ink syringe 1004 is connected to the bio-ink flow channel of the bioprinter nozzle 1001 through the bio-ink connection pipe 1015. A syringe pressure plate 1022 is disposed at the barrel outlet of the syringe. A pipe clamping block 1028 and a pipe fixing block 1020 are also disposed in front of the syringe to fix the bioprinter nozzle 1001, the bio-ink connection pipe 1015 and the medical adhesive connection pipe 1016, and the syringe pressure plate 1022 is pressed into the pipe clamping block 1028 and fixed by matched pins to prevent the syringe as a whole from being pulled backwards when the plunger is pulled.

The pipe clamping block 1028 is pressed on the pipe fixing block 1020 by the pipe clamping block pressing plate 1019 which moves vertically up and down relative to the pipe clamping block 1028. Both the pipe fixing block 1020 and the syringe mounting plate 1017 are secured to a movable base plate disposed on the lateral side thereof.

The pipe fixing block 1020 is provided with a slide groove on the lateral side, and a support bar 1021 is provided in the slide groove and can slide along the slide groove, and the support bar 1021 is provided in parallel with the bioprinter nozzle 1001, and a support plate 1027 is provided on the top of support bar 1021 in a clearance fit with the outer wall of the bioprinter nozzle 1001. The support bar 1021 and the support plate 1027 determine the movement direction of the bioprinter nozzle 1001, so that the bioprinter nozzle 1001 maintains straightness.

The artificial lumen mounting assembly 1030 includes two fixture blocks with semicircular slots. The lateral side of one fixture block is connected with the drive assembly 1009 and serves as a fixed fixture block. The other fixture block serves as a cover plate fixture block and is connected with the fixed fixture block by hinges. Each of the fixed fixture block and the cover plate fixture block is provided therein with a temperature control module 1013, which can regulate the temperature of the artificial lumen mounting cavity 1014. After combination of the two fixture blocks, the two semicircular slots are combined to form a cylindrical hollow cavity with the diameter slightly greater than the outer diameter of the bioprinter nozzle 1001 to act as the artificial lumen mounting cavity 1014. The length of the artificial lumen mounting cavity 1014 is slightly smaller than the length of the medical adhesive flow channel of the bioprinter nozzle 1001. The artificial lumen for the lumen tissue construct has an inner diameter slightly larger than the outer diameter of the bioprinter nozzle 1001 after the artificial lumen for the lumen tissue construct is mounted in the artificial lumen mounting cavity 1014.

The drive assembly 1009 includes a drive motor 1006, a screw rod 1008, a slider 1010, a connection plate 1011, and a sliding rail 1007. The screw rod 1008 is connected to the output shaft of the drive motor 1006, and the slider 1010 is provided on and movably engaged with the screw rod 1008. A segment of the slider 1010 is connected with the connection plate 1011, the sliding rail 1007 is provided below the connection plate 1011, which is connected with the fixed fixture block. The screw rod 1008 is driven by the drive motor 1006 to rotate to drive the slider 1010 to move on the screw rod 1008, and the slider 1010 during movement drives the connection plate 1011 to move on the sliding rail 1007, so that the artificial lumen mounting assembly 1030 is driven by the connecting plate 1011 to move.

A detection module 1012 is fixed at the top end of the artificial lumen mounting cavity 1014 of the artificial lumen mounting assembly 1030. The detection module 1012 includes a bracket and a camera fixed to the bracket, and the camera directly facing the upper portion of the artificial lumen mounting cavity 1014 is used for detecting whether the bioprinter nozzle 1001 reaches the initial position. The initial position may be the position where the medical adhesive ejection port of the bioprinter nozzle 1001 is aligned with the upper edge of the artificial lumen for the lumen tissue construct.

Both the artificial lumen mounting assembly 1030 and the detection module 1012 are connected with the drive assembly 1009 in a driving manner to move up and down on the sliding rail 1007.

In the bioprinter of this embodiment, the bioprinter nozzle 1001 and the artificial lumen mounting assembly 1030 can move relative to each other along the axial direction of the artificial lumen mounting cavity 1014, and during the movement, constant, uniform and simultaneous coating the inner wall of the artificial lumen for the lumen tissue construct with the medical adhesive and the bio-ink is performed. By providing the detection module 1012, it is possible to ensure that both the medical adhesive and the bio-ink are in a critical overflow state, so that the medical adhesive and the bio-ink can immediately leave the medical adhesive ejection port and the bio-ink ejection port at the start of the coating operation, ensuring that the overall coated length has no deviation.

Embodiments of the present disclosure further provide a lumen tissue construct printing method using the above bioprinter to print a lumen tissue construct. The artificial lumen for the lumen tissue construct is, for example, an artificial blood vessel.

The lumen tissue construct printing method includes the following steps:
filling printing material, including filling the feeding assembly 1005 with the printing material.

Two syringes, the medical adhesive syringe 1003 and the bio-ink syringe 1004, are filled with the corresponding printing materials and their respective plungers are left in an extended state. The bioprinter nozzle 1001 is connected with the two syringes, and then mounted to the pipe fixing block 1020, and the syringes are also synchronously mounted to the syringe mounting plate 1017. The pipe clamping block 1028 is then mounted to the pipe fixing block 1020. Then the movable base plate with the pipe fixing block 1020, the pipe clamping block 1028, the syringe mounting plate 1017, the syringes and the bioprinter nozzle 1001 is placed on the mounting base 1002 as a whole, and the syringes and the pipe clamping block 1028 are fixed by syringe clamping plate 1018 and the pipe clamping block pressing plate 1019, at which time the syringe plunger retaining tab 1024 is in the extended state. In the mounting process, the end disc of the plunger can be put into the gap between the syringe plunger retaining tab 1024 and the plunger retaining spring assembly 1029.

The artificial lumen for the lumen tissue construct is mounted, including mounting the artificial lumen in the artificial lumen mounting cavity 1014 of the artificial lumen mounting assembly 1030. The artificial lumen for the lumen tissue construct is placed in the artificial lumen mounting cavity and clamped and fixed.

The temperature of the artificial lumen mounting cavity 1014 is regulated. The temperature control module 1013 is set to a suitable temperature.

Initially positioning the bioprinter nozzle includes allowing the bioprinter nozzle 1001 to extend into the artificial lumen and be in an initial position. The drive assembly 1009 is controlled to drive the artificial lumen mounting assembly 1030 to move downward. When the artificial lumen mounting assembly 1030 contacts the support plate 1027, the support plate 1027 at the moment drives the support bar 1021 to move downward. The support plate 1027 can ensure that the bioprinter nozzle 1001 is coaxial with the artificial lumen for the lumen tissue construct when it contacts the latter, so that the bioprinter nozzle 1001 can smoothly enter into the lumen tissue construct and continue the relative movement until the top of the bioprinter nozzle 1001 extends out from the lumen tissue construct. When the detection module 1012 detects that the bioprinter nozzle 1001 reaches the initial position (e.g., the top of the bioprinter nozzle 1001 just extends out from the artificial lumen), the push-pull mechanism 1026 is controlled to push the medical adhesive and the bio-ink. After the camera monitors that there is an overflow of the medical adhesive and the bio-ink from the bioprinter nozzle 1001, the push-pull mechanism 1026 is suspended; and by pre-injecting the medical adhesive and the bio-ink into the bioprinter nozzle 1001 and monitoring the two by the camera, a pre-determination can be made as to whether each flow channel of the bioprinter nozzle 1001 is unobstructed before the start of printing.

Coating with printing material is performed. The drive assembly 1009 is controlled to moves in reverse, and when the bioprinter nozzle 1001 moves to a set position on the artificial lumen (e.g., a position where the medical adhesive ejection port is aligned with the upper edge of the artificial lumen), the push-pull mechanism 1026 is activated to push forward, so that the bioprinter nozzle 1001 squeezes out the medical adhesive and the bio-ink simultaneously. In the upward movement process of the artificial lumen along the bioprinter nozzle 1001, the medical adhesive is applied to the inner wall of the artificial lumen from top to bottom to form the medical adhesive layer, the bio-ink is also applied to the inner wall of the medical adhesive layer from top to bottom to process the medical adhesive layer and the bio-ink layer simultaneously, and the movement of the push mechanism stops until the set coated length is achieved.

The lumen tissue construct is taken out. The drive assembly 1009 continues to drive the artificial lumen mounting assembly 1030 to move upward, so that the bioprinter nozzle 1001 leaves the artificial lumen mounting cavity, and the cover plate fixture block is turned over to take out the lumen tissue construct with a biological construct layer (including the medical adhesive layer and the bio-ink layer).

After the operation is completed, the push-pull mechanism 1026 is pulled backward to drive the plungers of the syringes to move backward.

Through the above steps, coating with the medical adhesive and the bio-ink can be completed simultaneously on the inner wall of the artificial lumen for the lumen tissue construct, so that the biological construct layer prepared in the artificial lumen for the lumen tissue construct can be formed at a time to ensure the effect of adhering the bio-ink. In addition, it is possible to start coating from a specific position, and the printing material is pushed to the ejection port before coating the inner wall of an artificial lumen, and direct discharge can be achieved at the start of coating, without any delay in discharge.

By using the bioprinter nozzle, the bioprinter, and lumen tissue construct printing method of the embodiments of the present disclosure, it can achieve simultaneous coating with the medical adhesive and the bio-ink on the inner wall of the artificial lumen for the artificial biological construct such as a lumen tissue construct, and the biological construct layer is formed at a time, as long as a bioprinter nozzle and an artificial lumen mounting cavity meeting the requirements are adopted according to the length of an artificial biological construct to be prepared, thus a longer lumen tissue construct can be processed, which is conductive to avoiding problems such as decreased viscosity of the medical adhesive due to multiple printing of the medical adhesive and the bio-ink as the required lumen tissue construct is too long.

By using the bioprinter nozzle, the bioprinter and the lumen tissue construct printing method of the embodiments of the present disclosure, the lumen tissue construct greater than or equal to 30 cm in length can be prepared.

Finally, it should be noted that the above embodiments are only used to illustrate the technical solutions of the present disclosure, instead of limiting the same. Although the present disclosure has been described in detail with reference to preferred embodiments, those of ordinary skill in the art should understand that the specific embodiments of the present disclosure can be modified or a part of the technical features can be equivalently substituted, and all the modification and substitution should be compassed in the scope of the technical solutions claimed by the present disclosure.

## Claims

1. A bioprinter nozzle, comprising:
a bio-ink flow channel (P1) disposed inside the bioprinter nozzle;
a bio-ink ejection port (J1) in communication with the bio-ink flow channel (P1) and located on the lateral side of an ejection end of the bioprinter nozzle;
a medical adhesive flow channel (P2) disposed inside the bioprinter nozzle and isolated from the bio-ink flow channel (P1); and
a medical adhesive ejection port (J2) in communication with the medical adhesive flow channel (P2) and located on the lateral side of the ejection end, the medical adhesive ejection port (J2) and the bio-ink ejection port (J1) being arranged in a staggered manner along the axial direction of the bioprinter nozzle.

2. The bioprinter nozzle according to claim 1, wherein the bio-ink ejection port (J1) is closer to the top surface of the ejection end than the medical adhesive ejection port (J2) along the axial direction of the bioprinter nozzle.

3. The bioprinter nozzle according to claim 1 or 2, wherein
the bioprinter nozzle comprises a plurality of bio-ink ejection ports (J1) uniformly distributed along the circumference of the bioprinter nozzle; and/or
the bioprinter nozzle comprises a plurality of the medical adhesive ejection ports (J2) uniformly distributed along the circumference of the bioprinter nozzle or the medical adhesive ejection ports (J2) comprise annular ports arranged around the axial direction of the bioprinter nozzle.

4. The bioprinter nozzle according to any one of claims 1 to 3, comprising a medical adhesive scraping portion (M) disposed on the lateral side of the bioprinter nozzle, the medical adhesive scraping portion (M) being located between the bio-ink ejection port (J1) and the medical adhesive ejection port (J2) along the axial direction of the bioprinter nozzle.

5. The bioprinter nozzle according to claim 4, wherein the medical adhesive scraping portion (M) comprises an annular projection or brush head disposed on the lateral side of the bioprinter nozzle.

6. The bioprinter nozzle according to any one of claims 1 to 5, comprising:
a bio-ink nozzle body on which the bio-ink ejection port (J1) is disposed; and
a medical adhesive nozzle body disposed on the periphery of the bio-ink nozzle body, the medical adhesive ejection port (J2) being disposed on the medical adhesive nozzle body or between the bio-ink nozzle body and the medical adhesive nozzle body.

7. The bioprinter nozzle according to claim 6, wherein
the bio-ink flow channel (P1) is disposed in the bio-ink nozzle body; and
the medical adhesive flow channel (P2) is disposed between the medical adhesive nozzle body and the bio-ink nozzle body.

8. The bioprinter nozzle according to claim 7, wherein the bioprinter nozzle comprises a connecting fin connected between the bio-ink nozzle body and the medical adhesive nozzle body.

9. The bioprinter nozzle according to claim 6, wherein
the bio-ink flow channel (P1) is disposed in the bio-ink nozzle body;
the medical adhesive flow channel (P2) is disposed in the bio-ink nozzle body, and the side wall of the bio-ink nozzle body is provided with a communication port (N) in communication with the medical adhesive flow channel (P2); and
the medical adhesive ejection port (j2) comprises an annular port arranged around the axial direction of the bioprinter nozzle, which is formed by the bio-ink nozzle body and an end of the medical adhesive nozzle body close to the ejection end, and the communication port (N) is in communication with the annular ejection port.

10. The bioprinter nozzle according to claim 6, further comprising a nozzle mounting base, wherein
the bio-ink flow channel (P1) comprises a first bio-ink flow channel section (P11) disposed in the bio-ink nozzle body and a second bio-ink flow channel section (P12) disposed in the nozzle mounting base, or the bio-ink flow channel P1) is disposed in the nozzle mounting base; and
the medical adhesive flow channel (P2) comprises a first medical adhesive flow channel section (P21) disposed in the medical adhesive nozzle body and a second medical adhesive flow channel section (P22) disposed in the nozzle mounting base.

11. The bioprinter nozzle according to claim 10, wherein the medical adhesive flow channel (P2) further comprises a first annular buffer groove (G), through which the first medical adhesive flow channel section (P21) is in communication with the second medical adhesive flow (P22).

12. The bioprinter nozzle according to claim 11, wherein the first annular buffer groove (G) is formed between the medical adhesive nozzle body and the nozzle mounting base.

13. The bioprinter nozzle according to claim 10, wherein the medical adhesive flow channel (P2) comprises a second annular buffer groove (L) disposed between the medical adhesive ejection port (J2) and the first medical adhesive flow channel section (P21).

14. A bioprinter, comprising:
a bioprinter nozzle (1001) comprising the bioprinter nozzle according to any one of claims 1 to 13;
a feeding assembly (1005) connected with the bioprinter nozzle (1001) and configured to supply bio-ink to the bio-ink flow channel (P1) of the bioprinter nozzle (1001) and supply medical adhesive to the medical adhesive flow(P2); and
an artificial lumen mounting assembly (1030) comprising an artificial lumen mounting cavity (1014) for accommodating an artificial lumen for a lumen tissue construct, the artificial lumen mounting assembly (1030) being configured to be capable of reciprocating relative to the bioprinter nozzle (1001) along the axial direction of the artificial lumen mounting cavity (1014).

15. The bioprinter nozzle according to claim 14, wherein the feeding assembly (1005) comprises:
a medical adhesive syringe (1003) configured to supply medical adhesive to the medical adhesive flow channel (P2);
a bio-ink syringe (1004) configured to supply bio-ink to the bio-ink flow channel (PI);
a syringe fixing assembly configured to fix the medical adhesive syringe (1003) and the bio-ink syringe (1004); and
a push mechanism configured to be drivingly connected with a plunger of the medical adhesive syringe (1003) and a plunger of the bio-ink syringe (1004) to drive the two plungers.

16. The bioprinter nozzle according to claim 15, wherein the push mechanism comprises:
a syringe plunger retaining tab (1024) provided with a notch for placing an end disc of a plunger;
a plunger retaining spring assembly (1029) comprising an assembly housing (10291) having a spring mounting cavity (10294) therein, a guide post (10292) and a spring (10293), the guide post (10292) movably passing through the assembly housing (10291) and a middle part of the guide post (10292) being provided in the spring mounting cavity (10294), a first end of the guide post (10292) being connected with the syringe plunger retaining tab (1024), the middle part of the guide post (10292) having a protruding portion facing the radial outer side, and the spring (10293) being sleeved on the periphery of the guide post (10292) and provided between the inner wall of the spring mounting cavity (10294) close to the first end of the guide post (10292) and the protruding portion;
a spring pushing tab (1025) provided at the end of the plunger retaining spring assembly (1029) away from the plunger; and
a push-pull mechanism (1026) drivingly connected with the assembly housing (10291), and configured to drive the assembly housing (10291) to switch a second end of the guide post (10292) and the spring pushing tab (1025) between an abutted state and a separated state; wherein in the abutted state, a space for mounting the end disc is formed between the syringe plunger retaining tab (1024) and the plunger retaining spring assembly (1029); in the separated state, the plunger retaining spring assembly (1029) presses and fixes the end disc to the syringe plunger retaining tab (1024); and the push-pull mechanism (1026) is configured to drive the plunger by the plunger retaining spring assembly (1029).

17. The bioprinter according to any one of claims 14 to 16, comprising a drive assembly (1009) configured to drivingly connect with the artificial lumen mounting assembly (1030) to drive the artificial lumen mounting assembly (1030) to reciprocate along the axial direction of the artificial lumen mounting cavity (1014).

18. The bioprinter according to any one of claims 14 to 17, comprising a detection module (1012) configured to detect a position of the bioprinter nozzle (1001) relative to the artificial lumen mounting cavity (1014).

19. The bioprinter according to any one of claims 14 to 18, comprising a temperature control module (1013) configured to regulate the temperature of the artificial lumen mounting cavity (1014).

20. The bioprinter according to any one of claims 14 to 19, further comprising:
a support bar (1021) extending along the axial direction of the artificial lumen mounting cavity (1014) and movably disposed; and
a support plate (1027) disposed at the top end of the support bar (1021) and comprising a nozzle mounting port configured to be in clearance fit with the outer wall of the bioprinter nozzle (1001) to maintain the bioprinter nozzle (1001) mounted in the nozzle mounting port to be coaxial with the artificial lumen mounting cavity (1014).

21. A lumen tissue construct printing method, using the bioprinter according to any one of claims 14 to 20 to print a lumen tissue construct, comprising:
filling printing material, comprising filling the feeding assembly (1005) with the printing material;
mounting the artificial lumen for the lumen tissue construct, comprising mounting the artificial lumen in the artificial lumen mounting cavity (1014) of the artificial lumen mounting assembly (1030);
initially positioning a bioprinter nozzle, comprising allowing the bioprinter nozzle (1001) to extend into the artificial lumen and be in an initial position; and
coating with the printing material, comprising moving the artificial lumen mounting assembly (1030) relative to the bioprinter nozzle (1001) along the axial direction of the artificial lumen mounting cavity (1014), and supplying bio-ink to the bio-ink flow channel (P1) of the bioprinter nozzle (1001) and supplying medical adhesive to the medical adhesive flow channel (P2) while moving.

22. The lumen tissue construct printing method according to claim 21, further comprising regulating the temperature of the artificial lumen mounting cavity (1014) after mounting of the artificial lumen for the lumen tissue construct.
